# EUROPÄISCHE PATENTSCHRIFT

(11) **EP 4 288 770 B1**
(45) Veröffentlichungstag und Bekanntmachung des Hinweises auf die Patenterteilung: **03.07.2024**
(21) Anmeldenummer: 21703259.8
(22) Anmeldetag: 03.02.2021
(51) Int. Cl.: G01N 27/22, G01N 33/44, B29C 35/00

(54) **VORRICHTUNG UND VERFAHREN ZUR MESSUNG DES AUSHÄRTEVORGANGS EINES HÄRTBAREN MATERIALS MITTELS DIELEKTRISCHER SPEKTROSKOPIE**
DEVICE AND METHOD FOR MEASURING THE CURING PROCESS OF A CURABLE MATERIAL BY MEANS OF DIELECTRIC SPECTROSCOPY
DISPOSITIF ET PROCÉDÉ DE MESURE DU PROCESSUS DE DURCISSEMENT D'UN MATÉRIAU DURCISSABLE PAR SPECTROSCOPIE DIÉLECTRIQUE

(43) Veröffentlichungstag der Anmeldung: 13.12.2023
(73) Patentinhaber: IGB-tech GmbH, 67159 Friedelsheim (DE)
(72) Erfinder: WELLMANN, Stefanie, 67159 Friedelsheim (DE); GUTOWSKI, Rafael, 67159 Friedelsheim (DE); DOEKER, Andreas, 82256 Fuerstenfeldbruck (DE)
(74) Vertreter: Beyer, Andreas
(86) Internationale Anmeldenummer: PCT/EP2021/052527
(87) Internationale Veröffentlichungsnummer: WO 2022/167066

(56) Entgegenhaltungen:
- US-A- 5 635 845
- POLANSKÝ R ET AL: "Development of a measuring system for on-line in situ monitoring of composite materials manufacturing", COMPOSITES PART A: APPLIED SCIENCE AND MANUFACTURING, ELSEVIER, AMSTERDAM, NL, Bd. 90, 6. September 2016 (2016-09-06), Seiten 760-770, XP029737654, ISSN: 1359-835X, DOI: 10.1016/J.COMPOSITESA.2016.09.006
- TOMAS BLECHA ET AL: "Epoxy resin curing process evaluation based on signal frequency analysis from interdigital structure sensor", ELECTRONIC SYSTEM-INTEGRATION TECHNOLOGY CONFERENCE (ESTC), 2010 3RD, IEEE, PISCATAWAY, NJ, USA, 13. September 2010 (2010-09-13), Seiten 1-4, XP031806636, ISBN: 978-1-4244-8553-6
- FREISLEBEN JAROSLAV ET AL: "Monitoring device of resin curing process", 2016 39TH INTERNATIONAL SPRING SEMINAR ON ELECTRONICS TECHNOLOGY (ISSE), IEEE, 18. Mai 2016 (2016-05-18), Seiten 452-455, XP032956665, DOI: 10.1109/ISSE.2016.7563239 [gefunden am 2016-09-07]

## Beschreibung

Die vorliegende Erfindung betrifft eine Vorrichtung und ein Verfahren zur Messung des Aushärtevorgangs eines härtbaren Materials mittels dielektrischer Spektroskopie.

Härtbare Materialien kommen heutzutage in vielen technischen Bereichen zum Einsatz. Härtbare Materialien können beispielsweise als Klebstoffe, Lacke, Vergussmassen, Oberflächenbeschichtungen und Ähnliches dienen. Wie der Begriff "härtbare Materialien" bereits erkennen lässt, sind solche Materialien in ihrem Ausgangszustand fließfähig oder zumindest pastös, um am vorgesehenen Einsatzort in einer gewünschten Menge einfach applizierbar zu sein. Erst nach Initiierung einer Härtungsreaktion beginnt ein Aushärtevorgang, der je nach der verwendeten Materialzusammensetzung extrem schnell verlaufen kann (z.B. im Bereich von Millisekunden) oder auch sehr viel länger dauern kann, beispielsweise mehrere Stunden. In jedem Fall soll am Ende ein gewünschter Aushärtezustand erreicht werden, um diejenigen Eigenschaften zu gewährleisten, die das gehärtete Material haben soll.

Die chemische Basis härtbarer Materialien ist mannigfaltig. Härtbare Materialien können organische Materialien, anorganische Materialien und Mischungen aus organischen und anorganischen Materialien sein. Häufig werden etwa radikalisch härtende Acrylate, ungesättigte Polyester, Polyurethane, Epoxidharze oder auch Silan-haltige Reaktanden eingesetzt. Härtbare Materialien können aus einem einzelnen härtbaren Bestandteil oder einer Kombination aus mehreren Bestandteilen bestehen, von denen einer oder mehrere härtbar sind. Die Härtungsreaktion kann auf chemischen Wege, z.B. durch Zusammenführen und Vermischen zweier Komponenten, aber auch durch Energiezufuhr ausgelöst werden, beispielsweise durch Erwärmen und/oder Bestrahlen mit energiereichem Licht, insbesondere UV-Licht. Lagerstabile Gebinde härtbarer Materialien bestehen in der Regel aus niedermolekularen Bestandteilen, wie Monomeren, Initiatoren, Oligomeren, Stabilisatoren, Füllstoffen und gegebenenfalls weiteren Additiven. Erst nach Initiierung der Härtungsreaktion härtet eine gewünschte Materialmischung durch beispielsweise Polymerisation aus, wobei der Grad der Aushärtung nicht nur von der Materialmischung selbst, sondern auch davon abhängt, welche Bedingungen zur Initiierung der Härtungsreaktion verwendet werden und wie lange diese Bedingungen aufrechterhalten werden.

Eine besondere, zuvor bereits kurz angesprochene Ausführungsform härtbarer Materialien sind solche Materialien, bei denen die Härtungsreaktion durch Bestrahlung mit UV-Licht initiiert wird und die daraufhin extrem schnell zu einem Polymer aushärten. Aufgrund der sehr schnellen Härtungsreaktion werden solche Materialien gerne in Fertigungsprozessen eingesetzt, die einen hohen Automatisierungsgrad haben. Beispiele für solche hochautomatisierte Fertigungsprozesse sind Lackierverfahren beispielsweise im Automobil- oder Möbelbau, die Aufbringung von Schutzlackierungen, sowie Vergussanwendungen und Klebeanwendungen auf dem Gebiet der Elektronik und Elektrotechnik sowie der Medizintechnik, der Optik oder des Maschinenbaus. Weitere Einsatzbereiche für insbesondere strahlungshärtbare Materialien sind die Kennzeichnung und Beschriftung von Verpackungen, etwa in der Lebensmittel- oder Pharmaindustrie.

Unabhängig von der Art eines härtbaren Materials ist es vor allem bei einem industriellen Einsatz solcher härtbarer Materialien wichtig, eine Kontrolle über das nach dem Härtungsvorgang erhaltene Ergebnis zu haben. Bei der Härtung von Materialien ist es in der Regel wichtig und erwünscht, eine vollständige Härtung zu erzielen. Zwar versteht es sich, dass im Rahmen einer industriellen Prozessauslegung die relevanten Parameter zunächst definiert und aufeinander abgestimmt werden, insbesondere durch eine Auswahl eines geeigneten härtbaren Materials und der zur Initiierung und Aufrechterhaltung der Härtungsreaktion erforderlichen Bedingungen (z.B. Temperatur und Menge einer Wärmezufuhr, Wellenlänge und Intensität einer Bestrahlung, etc.), jedoch ist dadurch noch nicht garantiert, dass das im industriellen Alltag erzielte Resultat immer dem gewünschten Ergebnis entspricht. Beispielsweise können Abweichungen von der spezifizierten Materialzusammensetzung, eine schleichende Änderung der Initiierungsbedingungen der Härtungsreaktion und/oder Änderungen in der Beschaffenheit der mit einem härtbaren Material in Kontakt kommenden Bauteile dazu führen, dass sich das Resultat eines Aushärtevorgangs eines verwendeten härtbaren Materials in unerwünschter Weise verändert. Es ist deshalb wünschenswert, den Verlauf und insbesondere das Ergebnis einer Härtungsreaktion eines härtbaren Materials möglichst einfach, schnell und "in situ" bestimmen zu können, um bei einer Abweichung vom gewünschten Ergebnis möglichst schnell geeignete Gegenmaßnahmen ergreifen zu können.

Grundsätzlich ist es bereits seit langem bekannt, den Aushärteprozess eines härtbaren Materials durch eine dielektrische Analyse zu untersuchen. Eine solche dielektrische Analyse wird auch als dielektrische Spektroskopie oder Dielektrizitätsspektroskopie bezeichnet und basiert auf einer Messung der Wechselwirkung eines elektromagnetischen Wechselfeldes mit den Dipolmomenten und Ionen in dem zu untersuchenden härtbaren Material. Allgemein lässt sich sagen, dass im flüssigen oder jedenfalls ungehärteten Zustand eines härtbaren Materials die Dipole in dem Material eine höhere Beweglichkeit aufweisen und beim Anlegen eines elektrischen Wechselfeldes mehr schwingen, wohingegen in einem festen, mehr oder weniger gehärteten Zustand die Beweglichkeit und Schwingungsbereitschaft der Dipole deutlich geringer ist. Geräte zur dielektrischen Analyse des Härtungsverhaltens von beispielsweise Polymeren sind kommerziell beispielsweise von der Firma Netzsch Gerätebau GmbH erhältlich.

Aus der wissenschaftlichen Veröffentlichung von R. Polansky et al in der Zeitschrift COMPOSITES PART A: APPLIED SCIENCES AND MANUFACTURING, ELSEVIER, AMSTERDAM, NL, Bd. 90, 6. September 2016, Seiten 760 bis 770 mit dem Titel "Development of a measuring system for on-line in situ monitoring of composite materials manufacturing" ist eine Vorrichtung zur Messung des Aushärtevorgangs eines härtbaren Materials mittels dielektrischer Spektroskopie bekannt, die aufweist:
- eine mobile Sendeeinheit mit einem Sensor zum Inkontaktkommen mit einer Probe des härtbaren Materials, wobei der Sensor dazu ausgestaltet ist, ein Signal in Form eines elektrischen Wechselfeldes mit einer vorgegebenen Frequenz in die Probe einzukoppeln,
- eine von der Sendeeinheit physisch getrennte Empfangseinheit, die drahtlos mit der Sendeeinheit zu kommunizieren vermag und eine Auswerteeinrichtung enthält, die Änderungen der vorgegebenen Frequenz erfasst, die im Laufe eines Messvorgangs auftreten, wobei die Auswerteeinrichtung der Empfangseinheit dazu eingerichtet ist, auf der Basis der während eines Messvorgangs erfassten Frequenzänderungen einen Zustand einer im Wesentlichen gleichbleibenden Frequenz zu bestimmen und diesen Zustand über eine Anzeigeeinrichtung

Weitere Veröffentlichungen, die Verfahren zur Verfassung des Aushärtegrades eines härtbaren Polymermaterials diskutieren, sind T. Blecha et al: "Epoxy resin curing process evaluation based on signal frequency analysis from interdigital structure sensor" Electronic system-integration technology conference 3rd, IEEE, 2010 1-4 und J. Freisleben et al: "Monitoring device of resin curing process" 39th international spring seminar on electronics technology (ISSE), IEEE 2016 452-455.

Aus der US 5 635 845 A sind ein Verfahren zur Erfassung des Aushärtegrades eines härtbaren Polymermaterials während eines Vorhärtungszustandes und eine Vorrichtung zur Ausführung dieses Verfahrens bekannt. Die Vorrichtung weist einen lösbar mit einer Sendeeinheit verbundenen Sensor auf, dessen Oberfläche mit Elektroden versehen ist, auf die das zu messende härtbare Material aufgebracht wird.

Ein Charakteristikum bisher zur Verfügung stehender Vorrichtungen zur dielektrischen Analyse besteht darin, dass solche Vorrichtungen auf einer aufwändigen elektrischen Analyse beruhen, die eine Messung der Phasenverschiebung und eine Aufgliederung in ein sogenanntes Speicher- und Verlustmodul beinhaltet. Sie eignen sich daher bevorzugt für stationäre Messungen im Labor, zur ortsfest installierten Messung in Guss- oder Pressformen oder als kabelgebundene Sensorik in Labor- oder Produktionseinrichtungen.

Andere Analysevorrichtungen wiederum erfordern es, eine Probe eines beispielsweise im Rahmen eines Fertigungsprozesses eingesetzten härtbaren Materials aus dem Produktionsbereich in ein Labor zu bringen, in dem die Vorrichtung zur Analyse steht, und dort die Probe zu analysieren und die im Rahmen der Analyse erhaltenen Daten auszuwerten. Es besteht deshalb der Wunsch nach einer Vorrichtung und einem Verfahren, mit der bzw. dem es auf kostengünstige, einfache und schnelle Weise möglich ist, den Ablauf und insbesondere das Ergebnis einer Härtungsreaktion eines härtbaren Materials möglichst vor Ort, d.h. am Einsatzort des härtbaren Materials, unmittelbar und vorzugsweise in Echtzeit präziser verfolgen zu können.

Die vorgenannte Aufgabe wird erfindungsgemäß durch eine Vorrichtung mit den im Patentanspruch 1 angegebenen Merkmalen und ein Verfahren mit den im Patentanspruch 10 angegebenen Schritten gelöst.

Eine erfindungsgemäße Vorrichtung zur Messung des Aushärtevorgangs eines härtbaren Materials mittels dielektrischer Spektroskopie weist somit auf:
- eine mobile Sendeeinheit mit einem Sensor zum in Kontakt kommen mit einer Probe des härtbaren Materials, wobei der Sensor dazu ausgestaltet ist, ein Signal in Form eines elektrischen Wechselfeldes mit einer vorgegebenen Frequenz in die Probe einzukoppeln, und
- eine von der Sendeeinheit physisch getrennte Empfangseinheit, die drahtlos mit der Sendeeinheit zu kommunizieren vermag und eine Auswerteeinrichtung enthält, die Änderungen der vorgegebenen Frequenz erfasst, die im Laufe eines Messvorgangs auftreten. Erfindungsgemäß ist der Sensor der Sendeeinheit auf einer lösbar mit der Sendeeinheit verbindbaren Sensorplatine ausgebildet und die Auswerteeinrichtung der Empfangseinheit ist dazu eingerichtet, auf der Basis der während eines Messvorgangs erfassten Frequenzänderungen zumindest drei Zustände zu bestimmen:
   -- einen ersten Zustand fallender Frequenz, der mit einem Aufbringen der Probe des ungehärteten Materials auf den Sensor korrespondiert,
   -- einen zweiten Zustand einer im Wesentlichen gleichbleibenden ersten Frequenz, der einem abgeschlossenen Aufbringen der Probe des ungehärteten Materials auf den Sensor entspricht, und
   -- einen dritten Zustand einer im Wesentlichen gleichbleibenden zweiten Frequenz, der einem Erreichen eines gewünschten Aushärtezustandes des härtbaren Materials entspricht, wobei die gleichbleibende zweite Frequenz höher ist als die gleichbleibende erste Frequenz. Erfindungsgemäß ist ferner die Empfangseinheit mit einer Anzeigeeinrichtung zum Anzeigen der zumindest drei Zustände versehen und die Vorrichtung ist dazu ausgebildet, nach einer stattgefundenen Bestimmung des zweiten Zustands einen Härtungsvorgang des härtbaren Materials zu initiieren.

Die erfindungsgemäße Vorrichtung hat eine Reihe von Vorteilen. Aufgrund der von der Empfangseinheit physisch getrennten Sendeeinheit kann die Sendeeinheit klein und kompakt ausgestaltet werden und ermöglicht somit eine Verwendung in unmittelbarer Nähe eines Einsatzortes eines zu untersuchenden härtbaren Materials. Die Anordnung des Sensors auf einer lösbar mit der Sendeeinheit verbindbaren Sensorplatine gestattet es, auf eine schnelle und kostengünstige Art eine ganze Reihe von Messungen nacheinander vorzunehmen, indem für jede Messung jeweils nur eine neue Sensorplatine mit der Sendeeinheit verbunden wird, beispielsweise durch Einstecken der Sensorplatine in die Sendeeinheit. Eine solche Ausgestaltung ermöglicht es darüber hinaus, sehr langsame Härtungsreaktionen auf einfache Art zu messen, denn eine Sensorplatine, auf deren Sensor ein Probenmaterial aufgebracht worden ist, kann nach einem initialen Härtungsvorgang von der Sendeeinheit gelöst und zu einem späterem Zeitpunkt (Tage, Wochen, Monate oder sogar Jahre später) wieder mit der Sendeeinheit verbunden werden, um die Messung fortzuführen. Auf diese Weise können auch Veränderungen des Probenmaterials erfasst werden, zu denen es beispielsweise aufgrund von Alterungsprozessen im Laufe der Zeit kommen kann. Ferner kann der Sensor als Einwegsensor ausgeführt werden, ohne dadurch die Kosten pro Messung signifikant zu erhöhen, denn Platinen der genannten Art lassen sich sehr kostengünstig in großen Stückzahlen herstellen. Alternativ kann der Sensor wiederverwendbar sein. Ein weiterer Vorteil der erfindungsgemäßen Vorrichtung besteht darin, dass keine komplizierte Auswertung der Messung erforderlich ist, sondern lediglich die Frequenzänderungen ausgewertet werden, die ausgehend von dem mit einer vorgegebenen Frequenz in die Probe eingekoppelten Signal während einer Messung auftreten. Allein aus diesen Frequenzänderungen werden die zumindest drei Zustände bestimmt und durch die Anzeigeeinrichtung der Empfangseinheit angezeigt, die einen Benutzer der Vorrichtung darüber informieren, dass a) mit einem Aufbringen einer Probe des ungehärteten Materials auf den Sensor begonnen worden ist, b) das Aufbringen der Probe auf den Sensor abgeschlossen ist, und c) ein gewünschter Aushärtezustand des härtbaren Materials erreicht worden ist. In Summe ermöglicht die erfindungsgemäße Vorrichtung somit eine einfache, schnelle, genaue und dennoch kostengünstige Messung des Aushärtevorgangs eines härtbaren Materials und informiert einen Benutzer der Vorrichtung quasi in Echtzeit über den jeweiligen Stand einer Messung und deren Ergebnis.

Gemäß einer bevorzugten Ausgestaltung der erfindungsgemäßen Vorrichtung ist der Sensor Teil eines einen Kondensator enthaltenden Schwingkreises, dessen Frequenzänderung während des Messvorgangs erfasst wird. Insbesondere kann der Sensor Teil einer frei schwingenden Oszillatorschaltung sein und kann, wenn der Sensor selbst als Kondensator ausgestaltet ist, das frequenzbestimmende Glied der frei schwingenden Oszillatorschaltung sein. Die messtechnische Erfassung eines solchen Schwingkreises kann mittels einer UltraFast Comparator-Schaltung erfolgen und ist seit langem bekannt, weshalb hier auf eine genauere Erläuterung verzichtet wird. Auf dem Markt sind hierfür kosteneffiziente und ausgereifte Lösungen erhältlich. Alternativ kann eine Schaltungsvariante eines mit fester Frequenz angeregten Schwingkreises dazu verwendet werden, den Härtevorgang des zu untersuchenden härtbaren Materials messtechnisch zu erfassen. Gemessen wird dabei die Abweichung der sich im Mess-Schwingkreis einstellenden Resonanzfrequenz zur vorgegebenen Referenzfrequenz.

Bei der erfindungsgemäßen Vorrichtung ist die Sensorfläche diejenige Fläche, die zur Messung des Aushärtevorgangs mit dem härtbaren Material in Kontakt kommen muss. Zum Messen des Aushärtevorgangs wird eine Probe des zu untersuchenden härtbaren Materials auf die Sensorfläche aufgebracht und danach wird der Härtungsvorgang initiiert. Vor Beginn einer Messung ist die Sensorfläche leer und eine vorgegebene Frequenz, die an dem Sensor anliegt, bleibt zumindest im Wesentlichen konstant. Wenn die Probe des härtbaren Materials auf die Sensorfläche aufgebracht wird, steigt aufgrund der nunmehr auf der Sensorfläche befindlichen Flüssigkeit die Dielektrizitätszahl an, was zu einer Erhöhung der Kapazität des im Schwingkreis enthaltenen Kondensators und damit zu einer Verringerung der Frequenz des Schwingkreises führt. Diese Frequenzänderung wird von der erfindungsgemäßen Vorrichtung erfasst.

Nachdem das Probenmaterial vollständig auf den Sensor aufgebracht worden ist, steigt die Dielektrizitätszahl und damit auch die Kapazität des im Schwingkreis enthaltenen Kondensators nicht mehr weiter an, so dass die Frequenz des Schwingkreises sich auf eine im Wesentlichen gleichbleibende, erste Frequenz einpendelt.

Sobald der Härtungsvorgang initiiert worden ist, verringert sich aufgrund der zunehmenden Aushärtung des Probenmaterials die Beweglichkeit der in ihm enthaltenen Dipole, was gleichbedeutend mit einer Verringerung der Dielektrizitätszahl ist, die somit eine abnehmende Kapazität des im Schwingkreis enthaltenen Kondensators und damit eine Zunahme der Frequenz des Schwingkreises zur Folge hat. Auch diese Frequenzänderung wird von der erfindungsgemäßen Vorrichtung erfasst.

Wenn der Härtungsvorgang sich seinem Ende nähert und das Probenmaterial vollständig oder zumindest nahezu vollständig ausgehärtet ist, fällt die Dielektrizitätszahl und damit auch die Kapazität des in dem Schwingkreis enthaltenen Kondensators nicht mehr weiter ab, so dass sich die Frequenz des Schwingkreises auf eine im Wesentlichen gleichbleibende, zweite Frequenz einpendelt, die höher ist als die erste Frequenz.

Gemäß einer besonders bevorzugten Ausführungsform der erfindungsgemäßen Vorrichtung ist der Sensor als Kondensator ausgestaltet und kann der Kondensator des Schwingkreises sein, dessen Frequenzänderung während des Messvorgangs erfasst wird. Wenn der Sensor als Kondensator ausgestaltet ist, dann weist der Sensor vorzugsweise zwei zusammenwirkende Kammelektroden auf, die seine Sensorfläche definieren. Besonders bevorzugt können dabei die zusammenwirkenden Kammelektroden auf einer Oberseite der Sensorplatine ausgebildet sein, beispielsweise indem die zusammenwirkenden Kammelektroden als ineinander eingreifende Leiterbahnen auf der Oberseite der Sensorplatine ausgestaltet sind und zusammen eine rechteckige, quadratische oder auch runde Sensorfläche bilden.

Die erfindungsgemäße Vorrichtung erfasst die Frequenzänderungen während eines Messvorgangs vorzugsweise dadurch, dass während vorgegebener Zeitfenster die Schwingungsperioden des Schwingkreises pro Zeiteinheit gemessen werden. Unter dem Begriff "Schwingungsperioden" werden im Rahmen der vorliegenden Erfindung nicht nur sinus- oder andersförmige Schwingungen verstanden, sondern auch Pulse, denn es kann zur Messung des Aushärtevorgangs statt einer vorgegebenen Schwingungsfrequenz ebenso gut eine vorgegebene Pulsfrequenz in die Probe eingekoppelt werden.

Die Größe der genannten Zeitfenster, während derer die Schwingungsperioden pro Zeiteinheit erfasst werden, kann frei gewählt werden, ist jedoch von der vorgegebenen Frequenz abhängig, die in die Probe eingekoppelt wird. Die vorgegebene Frequenz, die in die Probe eingekoppelt wird, hängt wiederum von dem zu untersuchenden Probenmaterial und einer gewünschten Eindringtiefe des elektrischen Wechselfeldes in das Probenmaterial ab. Gemäß einer Ausführungsform der erfindungsgemäßen Vorrichtung beträgt die vorgegebene Frequenz etwa 9 MHz und die Zeitfenster, während derer die Schwingungsperioden pro Zeiteinheit erfasst werden, haben jeweils eine Länge im Bereich von ca. 50 bis 150 Millisekunden.

Während des Aushärtevorgangs entsteht aufgrund der dabei stattfindenden Kondensations- oder Polymerisationsvorgänge ein unter Umständen erhebliches Maß an Wärme. Um die Temperatur des Probenmaterials während des Aushärtevorgangs verfolgen zu können, ist die erfindungsgemäße Vorrichtung vorteilhaft mit einem Temperatursensor ausgestattet. Gemäß einer bevorzugten Ausgestaltung befindet sich dieser Temperatursensor auf der Sensorplatine und ist dem Sensor, genauer der Sensorfläche, zugeordnet. Besonders bevorzugt ist der Temperatursensor auf einer Rückseite der Sensorplatine in einem Bereich angeordnet, der auf der Oberseite der Sensorplatine von der Sensorfläche eingenommen wird.

Damit die Sendeeinheit drahtlos mit der Empfangseinheit kommunizieren kann, benötigt die Sendeeinheit eine Antenne. Diese Antenne ist gemäß einer vorteilhaften Ausgestaltung der erfindungsgemäßen Vorrichtung auf der Sensorplatine angeordnet, beispielsweise in Gestalt eines auf der Ober- und/oder Rückseite der Sensorplatine ausgebildeten elektrischen Leiters.

Wie bereits erläutert, kann das Initiieren des Härtungsvorgangs durch eine Bestrahlung mit Licht, insbesondere UV-Licht erfolgen. Vorteilhafte Ausgestaltungen der erfindungsgemäßen Vorrichtung sind daher mit einem UV-Sensor zur Erfassung einer UV-Bestrahlung der Probe versehen, wobei der UV-Sensor vorzugsweise Teil der Sendeeinheit ist und ferner so angebracht ist, dass er UV-Licht erfasst, welches auf den Sensor trifft, auf dem sich das Probenmaterial befindet. Mittels eines solchen UV-Sensors lassen sich als zusätzliche Information der Beginn und die Dauer einer UV-Bestrahlung exakt erfassen.

Die Anzeigeeinrichtung der erfindungsgemäßen Vorrichtung ist dazu in der Lage, die zumindest drei bestimmten Zustände anzuzeigen. Die Anzeigeeinrichtung kann beispielsweise ein mit der Vorrichtung in Verbindung stehender Bildschirm sein. In einer vorteilhaften Ausgestaltung der erfindungsgemäßen Vorrichtung besteht die Anzeigeeinrichtung lediglich aus drei LED, die insbesondere verschiedenfarbig sein können, um eine schnelle und eindeutige optische Erfassung der drei Zustände zu erleichtern. Beispielsweise kann der erste Zustand durch eine rote LED angezeigt werden, der zweite Zustand durch eine gelbe LED und der dritte Zustand durch eine grüne LED. Vorzugsweise befindet sich eine solche Anzeigeeinrichtung an der Empfangseinheit, jedoch kann alternativ oder auch zusätzlich die Sendeeinheit mit einer Anzeigeeinrichtung zur Anzeige der zumindest drei Zustände versehen sein.

Gemäß einer Weiterbildung der erfindungsgemäßen Vorrichtung ist diese nicht nur zur Messung des Aushärtevorgangs eines härtbaren Materials ausgebildet, sondern darüber hinaus zur Ansteuerung wenigstens einer Anlagenkomponente, beispielsweise einer Anlagenkomponente, die den Verlauf des Aushärtevorgangs beeinflusst. Bei bevorzugten Ausführungsformen der erfindungsgemäßen Vorrichtung ist deshalb die Empfangseinheit mit wenigstens einem Relais oder einem anderen Ansteuerungselement ausgestattet, das mit der Auswerteeinrichtung verbunden und dazu eingerichtet ist, eine Anlagenkomponente in Abhängigkeit wenigstens eines der zumindest drei bestimmten Zustände zu steuern. Beispielsweise kann eine solchermaßen ausgestattete erfindungsgemäße Vorrichtung das Initiieren des Härtungsvorgangs selbst starten, indem sie nach einer stattgefundenen Bestimmung des zweiten Zustands eine Lichtquelle zur Bestrahlung des Probenmaterials aktiviert. Viele weitere Ausgestaltungen sind für einen Fachmann unter Verwendung eines oder mehrerer Ansteuerungselemente ohne weiteres denkbar.

Die eingangs genannten Aufgaben werden erfindungsgemäß auch durch ein Verfahren zur Messung des Aushärtevorgangs eines härtbaren Materials mittels dielektrischer Spektroskopie gelöst, welches die folgenden Schritte aufweist:
- Einkoppeln eines Signals in Form eines elektrischen Wechselfeldes mit einer vorgegebenen Frequenz in eine Probe des härtbaren Materials und Ermitteln eines ersten Zustands fallender Frequenz, der mit einem Aufbringen der Probe des ungehärteten Materials auf einen Sensor zum Einkoppeln des Signals korrespondiert,
- Ermitteln eines zweiten Zustands einer im Wesentlichen gleichbleibenden ersten Frequenz, der einem abgeschlossenen Aufbringen der Probe des ungehärteten Materials auf den Sensor entspricht,
- Initiieren, wenn der zweite Zustand ermittelt worden ist, einer Härtungsreaktion des härtbaren Materials, und
- Ermitteln eines dritten Zustands einer im Wesentlichen gleichbleibenden zweiten Frequenz, der einem Erreichen eines gewünschten Aushärtezustandes des härtbaren Materials entspricht, wobei die gleichbleibende zweite Frequenz höher ist als die gleichbleibende erste Frequenz, und
- Anzeigen des jeweils ermittelten Zustands.

Erfindungsgemäß erfolgt ein Messen der Frequenz des elektrischen Wechselfeldes in Form von Schwingungsperioden oder auch Pulsen pro festgelegter Zeiteinheit.

Um eine gute Vergleichbarkeit verschiedener Messungen sicherzustellen, umfasst das erfindungsgemäße Verfahren vorzugsweise den Schritt des Justierens eines jeden Sensors auf einen gleichen Ausgangswert der Frequenz des elektrischen Wechselfeldes vor einem Aufbringen der Probe des ungehärteten Materials. Wichtig ist dabei nicht in erster Linie ein bestimmter Wert der Frequenz, sondern vielmehr ein gleicher Ausgangswert aller Sensoren, die zur Messung des Aushärtevorgangs eines bestimmten härtbaren Materials Verwendung finden sollen.

Um ein Auswerten der erfassten Frequenzänderungen zu erleichtern und eine genauere Zuordnung zu ermöglichen, umfassen vorteilhafte Ausgestaltungen des erfindungsgemäßen Verfahrens ein Messen einer Bestrahlungsdauer der Probe mit UV-Licht. Alternativ oder zusätzlich umfassen vorteilhafte Ausgestaltungen des erfindungsgemäßen Verfahrens ein Messen der Temperatur der Probe während der Dauer der Einkopplung des Signals in Form eines elektrischen Wechselfeldes in die Probe des härtbaren Materials.

Besonders bevorzugte Ausgestaltungen des erfindungsgemäßen Verfahrens umfassen den Schritt eines Messens einer Frequenzänderung eines als Kondensator ausgestalteten Sensors, der Teil eines Schwingkreises ist, zum Bestimmen des ersten, zweiten und dritten Zustands. Die Wirkungsweise eines solchen Sensors wurde zuvor im Zusammenhang mit der erfindungsgemäßen Vorrichtung bereits ausführlich erläutert und braucht deshalb hier nicht wiederholt zu werden.

Die erfindungsgemäße Vorrichtung und das erfindungsgemäße Verfahren eignen sich insbesondere zur Ermittlung des Polymerisationsverhaltens (zeitlicher Verlauf) unbekannter härtbarer Materialien bei gegebenen Aushärtebedingungen und zur vergleichenden Interpretation. Ferner sind die erfindungsgemäße Vorrichtung und das erfindungsgemäße Verfahren gut geeignet zur Ermittlung optimaler Aushärtungsparameter bei Verwendung verschiedener Initiierungsquellen (thermisch, chemisch oder Lichtbestrahlung, insbesondere mittels UV- oder IR-Licht unterschiedlicher Wellenlängen), zur Bestimmung einer thermischen Belastung des härtbaren Materials während seiner Aushärtung, zur Ermittlung einer notwendigen Schwellintensität zur Initiierung des Aushärtevorgangs und zur Ermittlung der zum Erreichen einer vollständigen Aushärtung notwendigen Bestrahlungsdosis oder Wärmemenge. Schließlich ermöglichen die erfindungsgemäße Vorrichtung und das erfindungsgemäße Verfahren die Überwachung und Einhaltung von Parametern, die für einen gegebenen Aushärtevorgang als passend definiert worden sind. Optional können beispielsweise Warnsignale ausgegeben werden, falls eine nicht mehr tolerable Abweichung von den definierten Parametern auftritt. Ferner können optional Eingriffe in die Prozesssteuerung erfolgen, beispielsweise eine Verlängerung und/oder Intensitätssteigerung einer UV-Bestrahlung, um einen gewünschten Aushärtezustand nach einer festgestellten Parameterabweichung wieder zu erreichen.

Ein Ausführungsbeispiel einer erfindungsgemäßen Vorrichtung und eines erfindungsgemäßen Verfahrens werden im Folgenden anhand der beigefügten, schematischen Figuren näher erläutert. Es zeigt:
- Figur 1: eine räumliche Darstellung einer Ausführungsform einer erfindungsgemäßen Vorrichtung mit einer Empfangseinheit und einer Sendeeinheit von schräg vorne und oben,
- Figur 2: eine der Ansicht aus Figur 1 entsprechende Ansicht der erfindungsgemäßen Vorrichtung von schräg hinten und oben,
- Figur 3a: eine Sensorplatine von oben,
- Figur 3b: die Sensorplatine aus Figur 3a von unten, und
- Figur 4: eine Messkurve, die bei einer Messung des Aushärtevorgangs eines härtbaren Materials mittels der erfindungsgemäßen Vorrichtung entsteht und auf der eine Anwendung des erfindungsgemäßen Verfahrens basiert.

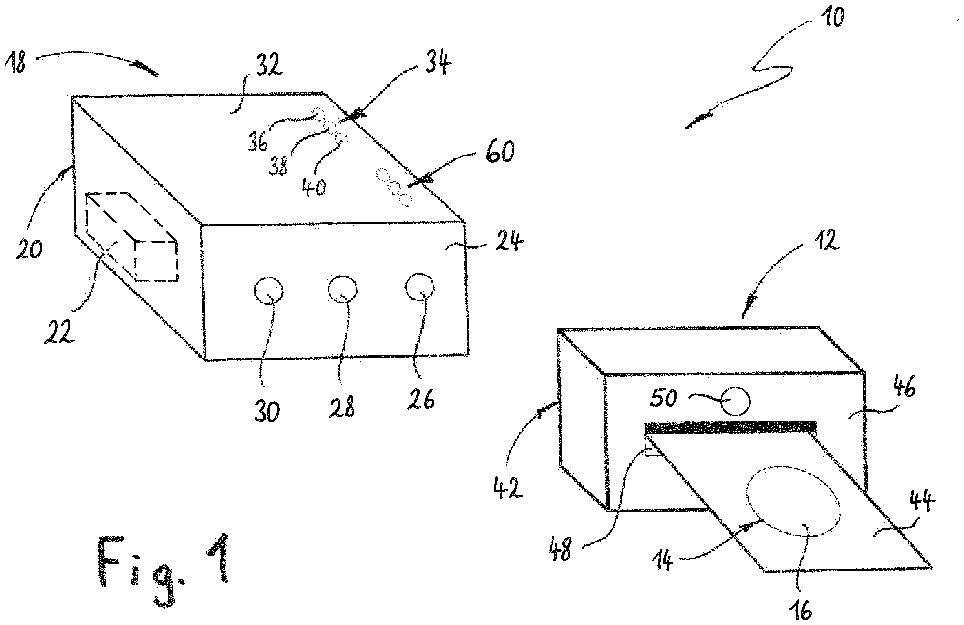

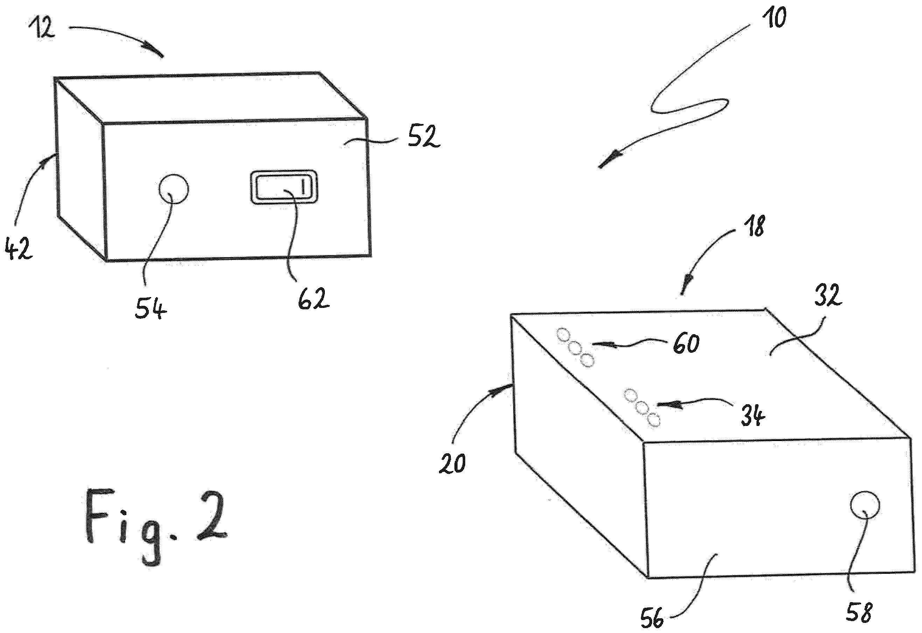

Die Figuren 1 und 2 zeigen eine allgemein mit 10 bezeichnete Vorrichtung zur Messung des Aushärtevorgangs eines härtbaren Materials mittels dielektrischer Spektroskopie. Die Vorrichtung 10 umfasst eine mobile Sendeeinheit 12 mit einem Sensor 14, der dazu ausgestaltet ist, ein Signal in Form eines elektromagnetischen Wechselfeldes mit einer vorgegebenen Frequenz in eine Probe des härtbaren Materials einzukoppeln. Damit der Sensor 14 das elektrische Wechselfeld in die Probe einkoppeln kann, wird Probematerial auf eine hier runde Sensorfläche 16 des Sensors 14 aufgebracht.

Die Vorrichtung 10 umfasst ferner eine von der Sendeeinheit 12 physisch getrennte Empfangseinheit 18 mit einem hier quaderförmigen Gehäuse 20, die drahtlos mit der Sendeeinheit 12 kommunizieren kann und in dem Gehäuse 20 eine mit gestrichelten Linien angedeutete Auswerteeinrichtung 22 enthält (nur in Fig. 1 gezeigt), die Frequenzänderungen erfasst, die im Laufe eines Messvorgangs auftreten.

An einer Vorderseite 24 des Gehäuses 20 sind ein Stromversorgungsanschluss 26, ein Steuerungsanschluss 28 und ein Datenkommunikationsanschluss 30 angeordnet. Alle Anschlüsse 26, 28 und 30 sind lediglich symbolisch dargestellt. Beispielsweise kann der Datenkommunikationsanschluss 30 als USB-Anschluss ausgebildet sein, so dass die Empfangseinheit 18 mit Hilfe eines USB-Kabels (nicht dargestellt) mit einem Bildschirm und/oder Computer (nicht dargestellt) verbunden werden kann. Der Stromversorgungsanschluss 26 kann als Buchse zur Aufnahme eines Netzkabels eines nicht dargestellten Netzteils ausgeführt sein. Der Steuerungsanschluss 28 kann eine Buchse sein, in die ein Steuerungskabel (nicht gezeigt) passt, welches die Empfangseinheit 18 mit einer zu steuernden Anlagenkomponente verbindet.

Auf einer Oberseite 32 des Gehäuses 20 weist die Empfangseinheit 18 eine Anzeigeeinrichtung 34 auf, die im gezeigten Ausführungsbeispiel aus drei LEDs 36, 38 und 40 besteht und deren Funktion später noch genauer erläutert wird.

Die mobile Sendeeinheit 12 besteht aus einem hier ebenfalls quaderförmigen Gehäuse 42 und einer den Sensor 14 mit seiner Sensorfläche 16 tragenden, hier rechteckigen Sensorplatine 44, die im gezeigten Ausführungsbeispiel als gedruckte Leiterplatte ausgebildet ist und in einen an der Vorderseite 46 des Gehäuses 42 ausgeformten Schlitz 48 gesteckt werden kann. Mittig oberhalb des Schlitzes 48 ist auf der Vorderseite 46 des Gehäuses 42 ein UV-Sensor 50 angeordnet, der UV-Licht erfassen kann, welches auf die Sensorfläche 16 und damit auf Probematerial auftrifft, welches sich auf der Sensorfläche 16 befindet.

Um die Sendeeinheit 12 netzstromunabhängig betreiben zu können, befindet sich im Gehäuse 42 der Sendeeinheit 12 eine aufladbare Batterie (nicht gezeigt), die den zum Betrieb der Sendeeinheit 12 notwendigen Strom liefert. Die aufladbare Batterie kann beispielsweise ein Lithiumionenakku sein oder auch ein oder mehrere sogenannte Supercaps. Um die im Gehäuse 42 befindliche Batterie aufladen zu können, ist auf der Rückseite 52 des Gehäuses 42 (siehe Figur 2) eine Ladeanschlussbuchse 54 vorgesehen, die über ein geeignetes Verbindungskabel (nicht gezeigt) mit einer an der Rückseite 56 des Gehäuses 20 der Empfangseinheit 18 vorgesehenen Ladebuchse 58 verbunden werden kann. Wenn die Sendeeinheit 12 mit der Empfangseinheit 18 unter Verwendung der Buchsen 54 und 58 verbunden ist, um die Batterie der Sendeeinheit 12 aufzuladen, kann der Ladezustand der Batterie anhand einer hier dreistufigen Ladezustandsanzeige 60 überwacht werden, die auf der Oberseite 32 des Gehäuses 20 der Empfangseinheit 18 vorhanden ist. Ergänzend kann eine gleich oder ähnlich ausgeführte Ladezustandsanzeige auch am Gehäuse 42 der Sendeeinheit 12 vorhanden sein (nicht dargestellt), um den Ladezustand der Batterie der Sendeeinheit 12 auch in einem Zustand prüfen zu können, in dem die Sendeeinheit 12 nicht zum Aufladen mit der Empfangseinheit 18 verbunden ist.

Auf der Rückseite 52 des Gehäuses 42 befindet sich ferner ein Schalter 62, der dazu dient, die Sendeeinheit 12 ein- und auszuschalten.

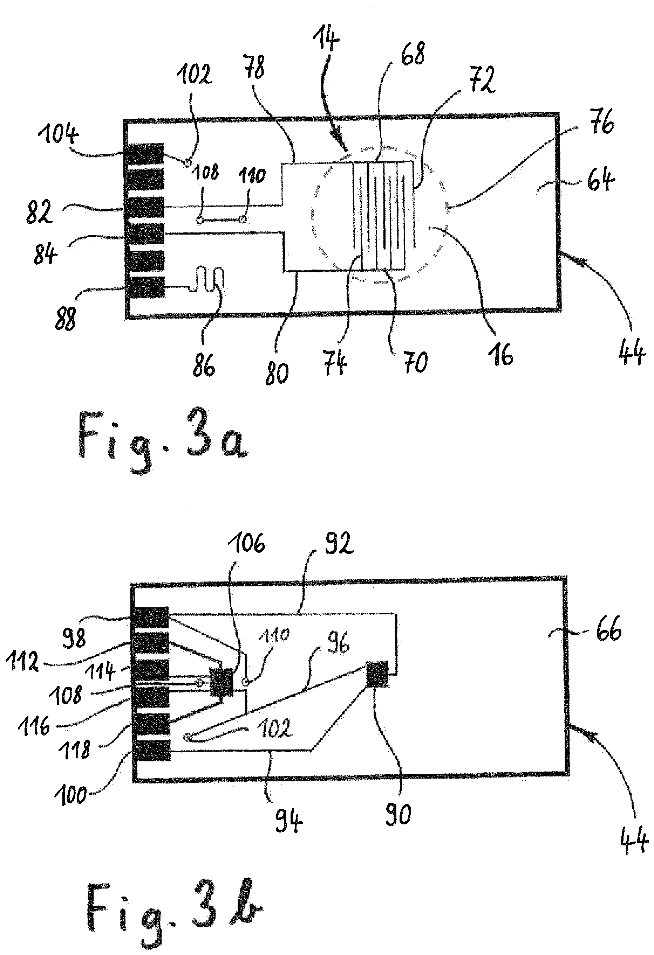

Unter Bezugnahme auf die Figuren 3a und 3b wird nun die Sensorplatine 44 genauer beschrieben, wobei Figur 3a eine Oberseite 64 und Figur 3b eine Unter- oder Rückseite 66 der Sensorplatine 44 zeigt.

Wie aus Figur 3a ersichtlich ist, befinden sich auf der Oberseite 64 der Sensorplatine 44 zwei aus elektrischen Leiterbahnen gebildete Kammelektroden 68 und 70, die ineinandergreifend angeordnet sind, sodass ihre Zähne 72 und 74 mit geringem Abstand untereinander abwechselnd aufeinander folgen. Die beiden Kammelektroden 68, 70 sind in Fig. 3a lediglich symbolisch dargestellt und füllen in Wahrheit die Sensorfläche 16 voll aus. Die Form und Anordnung der Kammelektroden 68, 70 ist somit maßgeblich für die hier runde Sensorfläche 16, deren Außenrand 76 so positioniert ist, dass sich die Kammelektroden 68, 70 gerade innerhalb der Sensorfläche 16 befinden. Jede Kammelektrode 68, 70 ist über eine Leiterbahn 78, 80 mit jeweils einem elektrischen Kontaktfeld 82, 84 auf der Oberseite 64 verbunden, welches an der in Figur 3a linken Schmalseite der Sensorplatine 44 ausgebildet ist.

Auf der Oberseite 64 der Sensorplatine 44 befindet sich ferner eine Antenne 86, die als mäanderförmige elektrische Leiterbahn ausgeführt und mit einem elektrischen Kontaktfeld 88 verbunden ist. Die Antenne 86 dient zur Drahtloskommunikation zwischen der Sendeeinheit 12 und der Empfangseinheit 18.

Auf der in Figur 3b dargestellten Unterseite 66 der Sensorplatine 44 befindet sich in einem von der Sensorfläche 16 überdeckten Bereich der Sensorplatine ein Temperatursensor 90, der über Leiterbahnen 92, 94 und 96 mit elektrischen Kontaktfeldern 98, 100 auf der Unterseite 66 und über eine Durchkontaktierung 102 mit einem elektrischen Kontaktfeld 104 auf der Oberseite 64 der Sensorplatine 44 in Verbindung steht. Der Temperatursensor 90 kann beispielsweise ein Thermistor vom Typ Pt100 sein und dient dazu, während der Messung eines Aushärtevorgangs eines härtbaren Materials, welches sich als Probe auf der Sensorfläche 16 befindet, die Temperatur des Probenmaterials zu messen.

Auf der Unterseite 66 der Sensorplatine 44 ist ferner ein integrierter Schaltkreis 106 angeordnet, der über Leiterbahnen elektrisch leitend mit dem Kontaktfeld 98 (über zwei Durchkontaktierungen 108 und 110) und mit weiteren Kontaktfeldern 112, 114, 116 und 118 verbunden ist und unter anderem dazu dient, die vorgegebene Frequenz zu kalibrieren, die der Sensor 14 in Gestalt eines elektromagnetischen Wechselfeldes in ein auf der Sensorfläche 16 befindliches Probematerial einkoppeln soll. Mittels des integrierten Schaltkreises 106 kann somit gewährleistet werden, dass jede Sensorplatine 44 eine gewünschte gleiche vorgegebene Frequenz in eine Probe einkoppelt, so dass mehrere mit verschiedenen Sensorplatinen 44 vorgenommene Messungen untereinander vergleichbar sind. Über die Kontaktfelder 112 und 118 kann die Sensorplatine 44 ein und ausgeschaltet werden. Der integrierte Schaltkreis 106 kann ferner dazu dienen, jede Sensorplatine 44 verwechselungssicher zu identifizieren, beispielsweise mittels eines in dem integrierten Schaltkreis 106 enthaltenen oder in ihn einprogrammierten Identifizierungscodes.

Die zuvor beschriebenen elektrischen Kontaktfelder auf der Oberseite 64 und Unterseite 66 der Sensorplatine 44 dienen zur Herstellung einer elektrisch leitenden Verbindung zwischen der Sensorplatine 44 und der Sendeeinheit 12, wenn die Sensorplatine 44 durch den Schlitz 48 in das Gehäuse 42 eingesteckt worden ist. In dem Gehäuse 42 befinden sich entsprechende, hier nicht gezeigte Gegenkontakte, die im eingesteckten Zustand der Sensorplatine 44 mit den Kontaktfeldern der Sensorplatine zusammenwirken.

Die wie beschrieben als gedruckte Leiterplatte ausgeführte Sensorplatine 44 kann zur Einmalverwendung vorgesehen sein und wird dann nach Durchführung einer Messung entsorgt. Es ist auch möglich, eine einmal verwendete Sensorplatine 44 erneut zu verwenden, jedoch muss dann sichergestellt sein, dass sich ein auf die Sensorfläche 16 aufgebrachtes Probenmaterial ohne eine Beschädigung der Sensorplatine 44 wieder von der Sensorfläche 16 lösen lässt. Dies kann beispielsweise durch eine geeignete Beschichtung der Sensorfläche 16 erreicht werden, die entweder ein endgültiges Anhaften des aufgebrachten Probenmaterials verhindert oder so hart ist, dass aufgebrachtes Probenmaterial wieder abgekratzt werden kann, ohne den darunterliegenden Sensor 14 zu beschädigen.

In der hier beschriebenen Ausführungsform bildet der durch die zwei zusammenwirkenden Kammelektroden 68, 70 definierte Sensor 14 einen Kondensator eines elektrischen Schwingkreises, dessen Frequenzänderung während des Messvorgangs von der Auswerteeinrichtung 22 erfasst wird. Der Sensor 14 ist dabei Teil einer frei schwingenden Oszillatorschaltung und stellt das frequenzbestimmende Glied der frei schwingenden Oszillatorschaltung dar. Die messtechnische Erfassung der Frequenzänderungen, zu denen es kommt, wenn ein zu untersuchendes härtbares Material auf der Sensorfläche 16 platziert und anschließend einem Härtungsvorgang unterzogen wird, erfolgt bei dem gezeigten Ausführungsbeispiel mittels einer an sich bekannten UltraFast Comparator-Schaltung (nicht dargestellt), die sich in der Sendeeinheit 12 befindet.

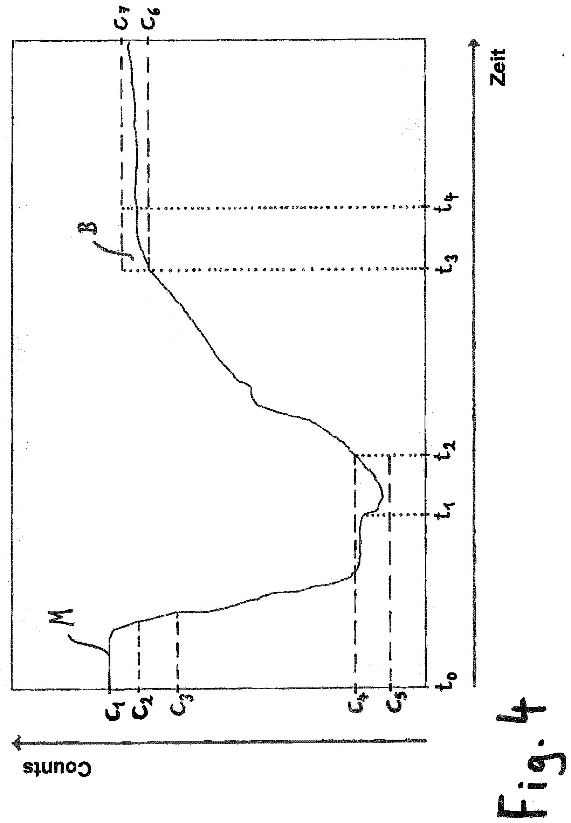

Die Durchführung einer Messung des Aushärtevorgangs eines härtbaren Materials wird im Folgenden unter Bezugnahme auf Figur 4 näher beschrieben, die eine Messkurve M zeigt, wie sie bei einer mittels der Vorrichtung 10 erfolgenden Messung des Aushärtevorgangs eines härtbaren Materials entsteht. In dem Diagramm gemäß Figur 4 ist die als Counts bezeichnete Anzahl von Schwingungsperioden pro Zeiteinheit des erwähnten Schwingkreises über dem zeitlichen Ablauf der Messung aufgetragen. Eine Messung beginnt zum Zeitpunkt t₀ mit einem leeren Sensor 14, d. h. auf der Sensorfläche 16 befindet sich keine zu untersuchende Probe. Die von der Auswerteeinrichtung 22 erfasste Anzahl an Counts hat zu Beginn einen Wert c₁, der der vorgegebenen Frequenz des elektromagnetischen Wechselfeldes entspricht, die am Sensor 14 anliegt. Diese vorgegebene Frequenz ist abhängig von dem zu untersuchenden härtbaren Material und der gewünschten Eindringtiefe des elektromagnetischen Wechselfeldes in das Probenmaterial und kann beispielsweise ca. 9 MHz betragen.

Sodann wird eine Probe des zu untersuchenden härtbaren Materials auf die Sensorfläche 16 aufgebracht. Um zu verhindern, dass das aufgebrachte Probenmaterial sich über die Sensorfläche 16 hinaus verteilt, kann der Außenrand 76 der Sensorfläche 16 leicht erhöht ausgebildet sein. Das Aufbringen des in der Regel flüssigen oder pastösen Probenmaterials auf die Sensorfläche 16 führt dazu, dass die Dielektrizitätszahl des als Kondensator fungierenden Sensors 14 und damit dessen Kapazität ansteigt, was wiederum ein Absinken der Frequenz des Schwingkreises nach sich zieht. Die Anzahl der von der Auswerteeinrichtung 22 erfassten Counts nimmt daher ab. In der Auswerteeinrichtung 22 sind zwei Count-Werte c₂ und c₃ festgelegt, für die gilt c₃ < c₂ < c₁. Wichtig ist dabei, dass der Wert c₂ ausreichend kleiner ist als der Wert c₁ und dass das Intervall zwischen c₂ und c₃ groß genug ist, um einen durch die Probenmaterialaufbringung hervorgerufenen Frequenzabfall eindeutig detektieren zu können. Das entlang der Messkurve durch die Werte c₂ und c₃ festgelegte Intervall entspricht dabei einem ersten Zustand fallender Frequenz, der mit einem Aufbringen des Probenmaterials auf den Sensor 14 korrespondiert. Das Erreichen bzw. Durchlaufen dieses ersten Zustandes wird durch die Anzeigeeinrichtung 34 angezeigt, indem die LED 36 aufleuchtet, die im gezeigten Ausführungsbeispiel eine rote LED ist.

Im Zuge des weiteren Aufbringens des Probenmaterials auf die Sensorfläche 16 sinkt der von der Auswerteeinrichtung 22 erfasste Count-Wert weiter ab, bis sich auf der Sensorfläche 16 eine materialspezifische Schichtdicke des Probenmaterials eingestellt hat, ab der ein weiteres Aufbringen von Probenmaterial zu keinem weiteren Absinken des Count-Wertes mehr führt, da eine jede vorgegebene Frequenz nur bis zu einer gewissen Tiefe in eine Probe eindringen kann. Da im Zuge des weiteren Aufbringens des Probenmaterials das durch die Werte c₂ und c₃ definierte Intervall nach unten verlassen worden ist, leuchtet die rote LED 36 nicht mehr.

Wenn genügend Probenmaterial auf die Sensorfläche 16 aufgebracht worden ist, stellt sich ein zweiter Zustand einer im Wesentlichen gleichbleibenden ersten Frequenz ein, der einem abgeschlossenen Aufbringen der Probe auf den Sensor 14 entspricht. In dem Diagramm gemäß Figur 4 ist dieser zweite Zustand durch ein Intervall definiert, welches durch die Count-Werte c₄ und c₅ festgelegt ist, wobei gilt c₅ < c₄. Die Werte c₄ und c₅ sind dabei so festgelegt, dass sich die im Wesentlichen gleichbleibende erste Frequenz, die einem zugehörigen Count-Wert entspricht, zwischen den Werten c₄ und c₅ befindet. Sobald der von der Auswerteeinrichtung 22 erfasste Count-Wert den Wert c₄ unterschreitet, wird dies als Erreichen des zweiten Zustandes gewertet und durch Aufleuchten der LED 38 signalisiert, die im gezeigten Ausführungsbeispiel eine gelbe LED ist. Wie Figur 4 zeigt, verläuft die Messkurve nun zunächst im Wesentlichen horizontal, d. h. es ist ein stationärer Zustand erreicht, der die abgeschlossene Aufbringung und Verteilung des Probenmaterials auf der Sensorfläche 16 charakterisiert.

Nun kann der Härtungsvorgang initiiert werden. Entsprechend der in Figur 4 wiedergegebenen Kurve geschieht dies zu einem Zeitpunkt t₁, beispielsweise durch eine Bestrahlung der das Probenmaterial tragenden Sensorfläche 16 mit UV-Licht. Zu Beginn des Aushärtevorgangs kann es aufgrund der durch die einsetzende Polymerisation des Probenmaterials erzeugte Temperaturerhöhung dazu kommen, dass der von der Auswerteeinrichtung 22 erfasste Count-Wert nochmals etwas absinkt (siehe Figur 4), weil das Probenmaterial aufgrund der Temperaturerhöhung noch etwas flüssiger wird und die Dipolbeweglichkeit in dem Probenmaterial kurzzeitig noch zunimmt. Der den zweiten Zustand mitdefinierende Wert es ist dabei so gewählt, dass der nach einem Initiieren des Härtungsvorgangs auftretende, niedrigste Count-Wert sich noch oberhalb des Werts c₅ und damit innerhalb des durch die Werte c₄ und c₅ definierten Intervalls befindet. Sollte eine durch die einsetzende Polymerisation auftretende Temperaturerhöhung des Probenmaterials zu groß werden, beispielsweise aufgrund einer zu intensiven oder zu langen Bestrahlung mit UV-Licht (oder allgemein ausgedrückt aufgrund einer zu harten Initiierung des Härtungsvorgangs), würde der Wert c₅ unterschritten werden und dadurch signalisieren, dass eine Überhitzung des Probenmaterials stattgefunden hat, die das Probenmaterial möglicherweise irreversibel geschädigt hat. Die Messung des Zeitverlaufs wird dann abgebrochen, was beispielsweise durch ein Blinken der gelben LED 38 angezeigt werden kann.

Eine ordnungsgemäße Initiierung des Härtungsvorgangs vorausgesetzt steigt die Messkurve nach eventuellem Durchlaufen des zuvor erläuterten lokalen Minimums wieder an und durchläuft erneut den Wert c₄, diesmal allerdings von unten nach oben. In dem Moment, in dem der von der Auswerteeinrichtung 22 erfasste Count-Wert den Wert c₄ in Richtung steigender Count-Werte durchläuft, erlischt die zuvor leuchtende, gelbe LED 38 und es wird zu diesem Zeitpunkt t₂ ein interner Timer (nicht dargestellt) gestartet, der zur Festlegung eines späteren durch Zeitpunkte t₃ und t₄ definierten zeitlichen Kontrollintervalls dient, in dem ein gewünschter Aushärtezustand erreicht worden sein soll.

Wie aus Figur 4 ersichtlich ist, steigt aufgrund der fortschreitenden Aushärtung (Polymerisation) des Probenmaterials der von der Auswerteeinrichtung 22 erfasste Count-Wert mehr oder weniger kontinuierlich an, bis ein dritter Zustand einer im Wesentlichen gleichbleibenden zweiten Frequenz erreicht wird, der einem gewünschten Aushärtezustand des härtbaren Materials entspricht. Dieser dritte Zustand ist durch Count-Werte c₆ und c₇ festgelegt, wobei gilt c₆ < c₇ und wobei in der Regel auch gilt c₇ < c₁. Die im Wesentlichen gleichbleibende zweite Frequenz entspricht dabei einem zugehörigen Count-Wert, der sich zwischen c₆ und c₇ befindet. Wie bereits ausgeführt, wird dieser dritte Zustand vorteilhaft nicht nur durch die Count-Werte c₆ und c₇, sondern darüber hinaus durch die Zeitwerte t₃ und t₄ definiert, so dass sich für den dritten Zustand das in Figur 4 dargestellte, durch die Count-Werte c₆ und c₇ und die Zeitwerte t₃ und t₄ begrenzte Kontrollfenster B ergibt, dessen Erreichen einen abgeschlossenen Aushärtevorgang mit einer definierten Aushärtungsqualität charakterisiert. Das Erreichen des dritten Zustandes wird durch Aufleuchten der LED 40 signalisiert, die im vorliegenden Ausführungsbeispiel eine grüne LED ist.

Aus dem beschriebenen Ablauf der Messung des Aushärtevorgangs eines härtbaren Materials ergeben sich eine Reihe von Vorteilen. Beispielsweise kann über den Steuerungsanschluss 28 die Initiierung des Härtungsvorgangs automatisch ausgelöst werden (durch entsprechende Ansteuerung beispielsweise der UV-Lichtquelle), sobald der Wert c₄ erstmals unterschritten wird. Voraussetzung dafür ist, dass sich der Wert c₄ nur wenig oberhalb desjenigen Count-Wertes befindet, der dem zweiten Zustand einer im Wesentlichen gleichbleibenden ersten Frequenz entspricht. Ferner ist es möglich, eine Energiezufuhr (Wärmezufuhr, Bestrahlung, etc.) dann automatisch zu beenden, sobald der Wert c₆ von unten her erreicht worden ist. Diese Möglichkeit gilt selbstverständlich nur für solche Systeme, in denen eine Energiezufuhr nicht nur zum Zwecke der Initiierung des Härtungsvorgangs, sondern auch während des Härtungsvorgangs stattfindet. Auch wäre es möglich, nach Erreichen der Zeit t₃ noch eine Nachbelichtung auszulösen, falls bis zu diesem Zeitpunkt der Wert c₆ noch nicht erreicht worden sein sollte.

Ein weiterer Vorteil des beschriebenen Ablaufs besteht darin, dass es der Härtungsverlauf (Polymerisationsverlauf) selbst ist, der zum Start des internen Timers führt. Dieser Start ist damit unabhängig von vorhergehenden Zeitverzögerungen, die beispielsweise durch den Ablauf der Dosierung des Probenmaterials auf die Sensorfläche 16 auftreten können.

## Patentansprüche

1. Vorrichtung (10) zur Messung des Aushärtevorgangs eines härtbaren Materials mittels dielektrischer Spektroskopie, wobei die Vorrichtung aufweist:
- eine mobile Sendeeinheit (12) mit einem Sensor (14) zum Inkontaktkommen mit einer Probe des härtbaren Materials, wobei der Sensor (14) dazu ausgestaltet ist, ein Signal in Form eines elektrischen Wechselfeldes mit einer vorgegebenen Frequenz in die Probe einzukoppeln, und
- eine von der Sendeeinheit (12) physisch getrennte Empfangseinheit (18), die drahtlos mit der Sendeeinheit (12) zu kommunizieren vermag und eine Auswerteeinrichtung (22) enthält, die Änderungen der vorgegebenen Frequenz erfasst, die im Laufe eines Messvorgangs auftreten,
wobei
- der Sensor (14) der Sendeeinheit (12) auf einer lösbar mit der Sendeeinheit verbindbaren Sensorplatine (44) ausgebildet ist,
- die Auswerteeinrichtung (22) der Empfangseinheit (18) dazu eingerichtet ist, auf der Basis der während eines Messvorgangs erfassten Frequenzänderungen zumindest drei Zustände zu bestimmen,
-- einen ersten Zustand fallender Frequenz, der mit einem Aufbringen der Probe des ungehärteten Materials auf den Sensor (14) korrespondiert,
-- einen zweiten Zustand einer im Wesentlichen gleichbleibenden ersten Frequenz, der einem abgeschlossenen Aufbringen der Probe des ungehärteten Materials auf den Sensor (14) entspricht, und
-- einen dritten Zustand einer im Wesentlichen gleichbleibenden zweiten Frequenz, der einem Erreichen eines gewünschten Aushärtezustandes des härtbaren Materials entspricht, wobei die gleichbleibende zweite Frequenz höher ist als die gleichbleibende erste Frequenz, und wobei
- die Empfangseinheit (18) eine Anzeigeeinrichtung (34) zum Anzeigen der zumindest drei Zustände aufweist, und
- die Vorrichtung (10) dazu ausgebildet ist, nach einer stattgefundenen Bestimmung des zweiten Zustands einen Härtungsvorgang des härtbaren Materials zu initiieren.

2. Vorrichtung nach Anspruch 1,
**dadurch gekennzeichnet, dass** der Sensor (14) Teil eines einen Kondensator enthaltenden Schwingkreises ist, dessen Frequenzänderung während des Messvorgangs erfasst wird.

3. Vorrichtung nach Anspruch 1 oder 2,
**dadurch gekennzeichnet, dass** der Sensor (14) als Kondensator ausgestaltet ist.

4. Vorrichtung nach Anspruch 3,
**dadurch gekennzeichnet, dass** der Sensor (14) zwei zusammenwirkende, eine Sensorfläche definierende Kammelektroden (68, 70) aufweist.

5. Vorrichtung nach Anspruch 4,
**dadurch gekennzeichnet, dass** die zwei zusammenwirkenden Kammelektroden (68, 70) auf einer Oberseite (64) der Sensorplatine (44) ausgebildet sind.

6. Vorrichtung nach einem der vorhergehenden Ansprüche,
**dadurch gekennzeichnet, dass** die Sensorplatine (44) einen dem Sensor (14) zugeordneten Temperatursensor (90) aufweist, der sich vorzugsweise auf einer Rückseite (66) der Sensorplatine (44) befindet.

7. Vorrichtung nach einem der vorhergehenden Ansprüche,
**dadurch gekennzeichnet, dass** die Sendeeinheit (12) einen UV-Sensor (50) zur Erfassung einer UV Bestrahlung der Probe aufweist.

8. Vorrichtung nach einem der vorhergehenden Ansprüche,
**dadurch gekennzeichnet, dass** die Anzeigeeinrichtung (34) drei LED (36, 38, 40) zur Anzeige der zumindest drei Zustände umfasst.

9. Vorrichtung nach einem der vorhergehenden Ansprüche,
**dadurch gekennzeichnet, dass** die Empfangseinheit (18) wenigstens ein mit der Auswerteeinrichtung (22) verbundenes Relais aufweist, das dazu eingerichtet ist, eine Anlagenkomponente in Abhängigkeit wenigstens eines der zumindest drei bestimmten Zustände zu steuern.

10. Verfahren zur Messung des Aushärtevorgangs eines härtbaren Materials mittels dielektrischer Spektroskopie, mit den Schritten:
- Einkoppeln eines Signals in Form eines elektrischen Wechselfeldes mit einer vorgegebenen Frequenz in eine Probe des härtbaren Materials,
- Ermitteln eines ersten Zustands fallender Frequenz, der mit einem Aufbringen der Probe des ungehärteten Materials auf einen Sensor (14) zum Einkoppeln des Signals korrespondiert,
- Ermitteln eines zweiten Zustands einer im Wesentlichen gleichbleibenden ersten Frequenz, der einem abgeschlossenen Aufbringen der Probe des ungehärteten Materials auf den Sensor (14) entspricht,
- Initiieren, wenn der zweite Zustand ermittelt worden ist, einer Härtungsreaktion des härtbaren Materials, und
- Ermitteln eines dritten Zustands einer im Wesentlichen gleichbleibenden zweiten Frequenz, der einem Erreichen eines gewünschten Aushärtezustandes des härtbaren Materials entspricht, wobei die gleichbleibende zweite Frequenz höher ist als die gleichbleibende erste Frequenz, und
- Anzeigen des jeweils ermittelten Zustands.

11. Verfahren nach Anspruch 10,
**gekennzeichnet durch** ein Messen der Frequenz des elektrischen Wechselfeldes in Form von Schwingungsperioden oder Pulsen pro festgelegter Zeiteinheit.

12. Verfahren nach Anspruch 10 oder 11,
**dadurch gekennzeichnet, dass** vor einem Aufbringen der Probe des ungehärteten Materials auf den Sensor jeder Sensor (14), der zur Messung des Aushärtevorgangs eines bestimmten härtbaren Materials Verwendung findet, auf einen gleichen Ausgangswert der Frequenz des elektrischen Wechselfeldes justiert wird.

13. Verfahren nach einem der Ansprüche 10 bis 12,
**gekennzeichnet durch** ein Messen einer Bestrahlungsdauer der Probe mit UV-Licht.

14. Verfahren nach einem der Ansprüche 10 bis 13,
**gekennzeichnet durch** ein Messen der Temperatur der Probe während der Dauer der Einkopplung des Signals in Form eines elektrischen Wechselfeldes in die Probe des härtbaren Materials.

15. Verfahren nach einem der Ansprüche 10 bis 14,
**gekennzeichnet durch** ein Messen einer Frequenzänderung eines als Kondensator ausgestalteten Sensors (14), der Teil eines Schwingkreises ist, zum Bestimmen des ersten, zweiten und dritten Zustands.

## Claims

1. A device (10) for measuring the curing process of a curable material by means of dielectric spectroscopy, wherein the device comprises:
- a mobile transmission unit (12) with a sensor (14) for coming into contact with a sample of the curable material, wherein the sensor (14) is designed to inject a signal in the form of an alternating electric field with a predetermined frequency into the sample, and
- a receiving unit (18), which is physically separate from the transmission unit (12), is configured to communicate wirelessly with the transmission unit (12) and contains an analysis device (22), which detects changes in the predetermined frequency that occur in the course of a measurement process,
wherein
- the sensor (14) of the transmission unit (12) is formed on a sensor circuit board (44) that is adapted to be detachably connected to the transmission unit,
- the analysis device (22) of the receiving unit (18) is adapted to determine, on the basis of frequency changes detected during a measurement process, at least three states,
-- a first state of decreasing frequency, which corresponds to a sample of the uncured material being applied to the sensor (14),
-- a second state of a substantially constant first frequency, which corresponds to a completed application of the sample of uncured material to the sensor (14), and
-- a third state of a substantially constant second frequency, which corresponds to a desired curing state of the curable material being reached, wherein the constant second frequency is higher than the constant first frequency, and wherein
- the receiving unit (18) has a display device (34) for displaying the at least three states, and
- the device (10) is configured to initiate, once determination of the second state has taken place, a curing process of the curable material.

2. The device according to Claim 1, **characterized in that** the sensor (14) is part of a resonant circuit containing a capacitor, the frequency change of which circuit is detected during the measurement process.

3. The device according to Claim 1 or 2, **characterized in that** the sensor (14) is configured as a capacitor.

4. The device according to Claim 3, **characterized in that** the sensor (14) has two cooperative comb electrodes (68, 70) defining a sensor surface.

5. The device according to Claim 4, **characterized in that** the two cooperative comb electrodes (68, 70) are formed on an upper side (64) of the sensor circuit board (44).

6. The device according to any one of the preceding claims, **characterized in that** the sensor circuit board (44) has a temperature sensor (90) associated with the sensor (14), which temperature sensor is preferably located on a rear side (66) of the sensor circuit board (44).

7. The device according to any one of the preceding claims, **characterized in that** the transmission unit (12) has a UV sensor (50) for detecting UV irradiation of the sample.

8. The device according to any one of the preceding claims, **characterized in that** the display device (34) comprises three LEDs (36, 38, 40) for displaying the at least three states.

9. The device according to any one of the preceding claims, **characterized in that** the receiving unit (18) has at least one relay connected to the analysis device (22), which relay is designed to control a system component depending on at least one of the at least three determined states.

10. A method for measuring the curing process of a curable material by means of dielectric spectroscopy, comprising the steps:
- injecting a signal in the form of an alternating electric field with a predetermined frequency into a sample of the curable material,
- determining a first state of decreasing frequency, which corresponds to a sample of the uncured material being applied to a sensor (14) for injecting the signal,
- determining a second state of a substantially constant first frequency, which corresponds to a completed application of the sample of uncured material to the sensor (14),
- initiating, when the second state has been determined, a curing reaction of the curable material, and
- determining a third state of a substantially constant second frequency, which corresponds to a desired curing state of the curable material being reached, wherein the constant second frequency is higher than the constant first frequency, and
- displaying the respectively determined state.

11. The method according to Claim 10, **characterized by** measuring the frequency of the alternating electric field in the form of oscillation periods or pulses per fixed unit of time.

12. The method according to Claim 10 or 11, **characterized in that** prior to application of the sample of uncured material to the sensor, each sensor (14) that is used for measuring the curing process of a given curable material is calibrated to an identical initial value of the frequency of the alternating electric field.

13. The method according to any one of Claims 10 to 12, **characterized by** measuring an irradiation duration of the sample with UV light.

14. The method according to any one of Claims 10 to 13, **characterized by** measuring the temperature of the sample during the duration of the injection of the signal in the form of an alternating electric field into the sample of curable material.

15. The method according to any one of Claims 10 to 14, **characterized by** measuring a frequency change of a sensor (14) configured as a capacitor, which sensor is part of a resonant circuit, to determine the first, second and third state.

## Revendications

1. Dispositif (10) de mesure du processus de durcissement d'un matériau durcissable par spectroscopie diélectrique, le dispositif comprenant:
- une unité d'émission mobile (12) doté d'un capteur (14) pour entrer en contact avec un échantillon du matériau durcissable, le capteur (14) étant conçu pour coupler un signal sous la forme d'un champ électrique alternatif à une fréquence prédéterminée dans l'échantillon, et
- une unité de réception (18) physiquement séparée de l'unité d'émission (12), qui peut communiquer sans fil avec l'unité d'émission (12) et qui contient un dispositif d'évaluation (22) qui détecte des modifications de la fréquence prédéterminée qui apparaissent au cours d'une opération de mesure,
dans lequel
- le capteur (14) de l'unité d'émission (12) est formé sur une platine de capteur (44) pouvant être reliée de manière amovible à l'unité d'émission,
- le dispositif d'évaluation (22) de l'unité de réception (18) est conçu pour déterminer au moins trois états sur la base des modifications de fréquence au cours de l'opération de mesure,
-- un premier état de fréquence décroissante qui correspond à une application de l'échantillon de matériau non durci sur le capteur (14),
-- un deuxième état d'une première fréquence sensiblement constante, qui correspond à une application achevée de l'échantillon du matériau non durci sur le capteur (14), et
-- un troisième état d'une deuxième fréquence sensiblement constante qui correspond à l'atteinte d'un état de durcissement souhaité du matériau durcissable, la deuxième fréquence constante étant supérieure à la première fréquence constante, et dans lequel
- l'unité de réception (18) présente un dispositif d'affichage (34) pour afficher les au moins trois états, et
- le dispositif (10) est conçu pour initier un processus de durcissement du matériau durcissable après qu'une détermination du deuxième état a eu lieu.

2. Dispositif selon la revendication 1,
**caractérisé en ce que** le capteur (14) fait partie d'un circuit oscillant contenant un condensateur dont la variation de fréquence est détectée pendant l'opération de mesure.

3. Dispositif selon la revendication 1 ou 2,
**caractérisé en ce que** le capteur (14) est conçu sous la forme de condensateur.

4. Dispositif selon la revendication 3,
**caractérisé en ce que** le capteur (14) comprend deux électrodes en peigne (68, 70) coopérant entre elles et définissant une surface de détection.

5. Dispositif selon la revendication 1,
**caractérisé en ce que** les deux électrodes en peigne (68, 70) coopérant entre elles sont disposées sur le côté supérieur (64) de la platine de capteur (44).

6. Dispositif selon l'une quelconque des revendications précédentes,
**caractérisé en ce que** la platine de capteur (44) comprend un capteur de température (90) associé au capteur (14) qui se trouve de préférence sur une face arrière (66) de la platine de capteur (44).

7. Dispositif selon l'une quelconque des revendications précédentes,
**caractérisé en ce que** l'unité d'émission (12) comprend un capteur UV (50) pour détecter un rayonnement UV de l'échantillon.

8. Dispositif selon l'une quelconque des revendications précédentes,
**caractérisé en ce que** le dispositif d'affichage (34) comprend trois DEL (36, 38, 40) permettant d'afficher les au moins trois états.

9. Dispositif selon l'une quelconque des revendications précédentes,
**caractérisé en ce que** l'unité d'émission (18) comprend au moins un relais relié au dispositif d'évaluation (22) qui est conçu pour commander un composant d'installation en fonction d'au moins l'un des au moins trois états déterminés.

10. Procédé (10) de mesure du processus de durcissement d'un matériau durcissable par spectroscopie diélectrique, comprenant les étapes:
- le couplage d'un signal sous la forme d'un champ électrique alternatif à une fréquence prédéfinie dans un échantillon du matériau durcissable,
- la détermination d'un premier état de fréquence décroissante qui correspondant à une application de l'échantillon du matériau durcissable sur un capteur (14) pour coupler le signal,
- - la détermination d'un deuxième état d'une première fréquence sensiblement constante, qui correspond à une application achevée de l'échantillon du matériau non durci sur le capteur (14),
- le lancement, lorsque le second état a été déterminé, d'une réaction de durcissement du matériau durcissable, et
- - la détermination d'un troisième état d'une deuxième fréquence sensiblement constante qui correspond à l'atteinte d'un état de durcissement souhaité du matériau durcissable, la deuxième fréquence constante étant supérieure à la première fréquence constante, et
- l'affichage de l'état respectif déterminé.

11. Procédé selon la revendication 10,
**caractérisé par** une mesure de la fréquence du champ électrique alternatif sous la forme de périodes d'oscillation ou d'impulsions par unité de temps déterminée.

12. Procédé selon la revendication 10,
**caractérisé en ce que**, avant une application de l'échantillon du matériau durcissable sur le capteur, chaque capteur (14) qui est utilisé pour mesurer le processus de durcissement d'un matériau durcissable déterminé, est réglé à une même valeur de sortie de la fréquence du champ électrique alternatif.

13. Procédé selon l'une quelconque des revendications 10 à 12,
**caractérisé par** une mesure d'une durée de rayonnement de l'échantillon avec la lumière UV.

14. Procédé selon l'une quelconque des revendications 10 à 13,
**caractérisé par** une mesure de la température de l'échantillon pendant la durée du couplage du signal sous la forme d'un champ électrique alternatif dans l'échantillon du matériau durcissable.

15. Procédé selon l'une quelconque des revendications 10 à 14,
**caractérisé par** une mesure d'une modification de fréquence d'un capteur (14) conçu sous la forme de condensateur, qui fait partie d'un circuit d'oscillation pour déterminer le premier, deuxième et troisième état.
